# EUROPEAN PATENT APPLICATION

(11) **EP 3 546 486 A1**
(43) Date of publication of application: **02.10.2019**
(21) Application number: 18164049.1
(22) Date of filing: 26.03.2018
(51) Int. Cl.: C08F 8/24, C08F 8/00, C08F 8/32, C08F 12/36, C08F 2/06, C08F 2/18, C08J 9/28, C08L 25/02, C07K 1/04, C08F 212/36, C08F 8/26, C08F 2/20

(54) **METHOD OF SOLID-STATE PEPTIDE SYNTHESIS USING A POLYMERIC SUPPORT**

(71) Applicant: Institute of Chemical Process Fundamentals of the CAS, v. v. i., 16502 Prague 6 - Suchdol (CZ); Università degli Studi di Padova, 35122 Padova (IT)
(72) Inventor: Jerabek, Karel, 18200 Praha 8 (CZ); Hankova, Libuse, 16000 Praha 6 - Vokovice (CZ); Holub, Ladislav, 10600 Praha 10 (CZ); Zecca, Marco, 35020 Masera di Padova (IT); Centomo, Paolo, 35030 Selvazzano Dentro (IT); Trovo, Daniele, 35010 Limena (IT); Leder, Riccardo, 36030 Zugliano (IT); Biondi, Barbara, 30171 Venezia (IT)
(74) Representative: Hartvichova, Katerina

(57) **Abstract**

The present invention provides a method of solid-state synthesis of peptides comprising the steps of
- providing a polymeric solid-state synthesis support,
- attaching a first amino acid to said support to form the first amino acid molecule of the desired peptide chain,
- attaching additional amino acids to form the desired peptide chain, characterized in that the polymeric solid-state synthesis support is a styrenic polymer having porosity 60 - 90 % and medium pore diameter ranging from 10 to 80 nm, said styrenic polymer being functionalized with chloromethyl and/or benzyloxybenzyl alcohol and/or amino moieties.

Furthermore, a functionalized styrenic polymer and its use in said method are claimed.

## Description

### Field of Art

The present invention relates to a method of solid-state peptide synthesis and to a novel polymeric support suitable for use in such a method.

### Background Art

Multi-step syntheses, like e.g. synthesis of peptides are advantageously performed using insoluble, solid polymer support to which the synthesized molecules are attached. In this arrangement, it is possible to achieve the isolation and purification of the intermediates after each individual synthesis step by simple filtration. After the final synthesis step, the product is split from the polymer support and recovered. In order to achieve an acceptable yield of the final product in such a multi-step synthesis, each step must be performed with a high yield, hence it is important to achieve high selectivity in each synthesis step. This requires minimization of the steric effects of the polymer backbone on the course of the reactions. The success of this strategy therefore depends to a great deal on properties of the polymer support. The morphology of the polymer in the working state must allow very good accessibility of the reactants to the reaction centers. Starting from the break-through invention of the solid-state synthesis of peptides by Merrifield [Merrifield, R. B.: J. Am. Chem. Soc. 1963, 85, 2149], for achieving this goal, low-crosslinked gel-type polymers swelling in the reaction environment have been used as the supports. The conditions of the polymer-supported synthesis, especially the reaction solvent, must be selected to ensure adequate polymer swelling. Generally, in solution, solvated molecules are able to move around and collide with one another. If the geometry and electrostatics of one of these collisions fulfills certain requirements imposed by the nature of the species involved, a new chemical bond is formed and a reaction is said to have occurred. The role of the solvent is to permit these collisions to occur by providing a kinetic medium for the molecules involved and create an electrostatic environment matching the reaction polarity. The environment of solid-phase organic reactions performed using gel-type polymer supports is the swollen polymer gel: it acts as the reaction medium and its properties are controlled by the nature of the polymer network [Vaino, A. R.; Janda, K. D.: J. Comb. Chem. 2000, 2, 579]. The classical polymer supports proposed by Merrifield, chloromethylated low-crosslinked co-polymers of styrene and divinylbenzene, are hydrophobic and in the synthesis of predominantly hydrophilic products like e.g. peptides they may not be quite optimal. It has been proven that with the purely styrenic polymer support the rate of incorporation of particular amino acids decreases, especially with increased peptide length [Martin F. G., Albericio F., Chem. Today 26(4) Supplement p. 26, 2008]. For the purpose of solving these intrinsic problems, various attempts have been made to modify the nature of the polymer backbone of the support by incorporation of acrylamide or polyethylene glycol moieties (e.g. Champion II®, ArgoGel®, CLEAR®, Tantagel®, NovaPeg® or ChemMatrix®). However, even in these more advanced polymer supports, the accessibility of the supported reaction centers depends on the polymer swelling and the selection of the reaction solvent is not polymer-independent. On the other hand, conventional rigid, permanently porous polymer supports with accessibility independent of their swelling have disadvantages, such as their low loading capacity and their limited accessibility due to low porosity and small pore sizes. This is the result of their morphology in which pores are created as spaces within clusters of polymer nanoparticles through macrosyneretic precipitation of the polymer within continuous solvent phase during polymerization in the presence of porogenic solvents. With this texture, surface area in the order of hundreds m²/g, required for an acceptable loading capacity, can be achieved only with pore sizes as low as a few nm, in which steric hindrances would be too high for efficient solid-state syntheses.
The present invention aims at providing a solid-state peptide synthesis support having all required properties without the need for compromising on some parameters to achieve suitable values of other parameters. The invention aims at providing a solid-state support having a rigid backbone, which does not change its volume with swelling, while not sterically hindering the synthesis of peptide chain and not significantly limiting the length of chains due to sterical hindrances. The solid-state support should also be compatible with various reaction solvents to allow for versatile solutions to peptide synthesis requirements.

### Disclosure of the Invention

An object of this invention is to provide a method of solid-state synthesis of peptides comprising the steps of
- providing a polymeric solid-state synthesis support,
- attaching a first amino acid to said support to form the first amino acid molecule of the desired peptide chain,
- attaching additional amino acids to form the desired peptide chain,
wherein the polymeric solid-state synthesis support is a styrenic polymer having porosity 60 to 90 %, and medium pore diameter ranging from 10 to 80 nm, said styrenic polymer being functionalized with chloromethyl and/or benzyl-oxy-benzyl alcohol and/or amino moieties.

Such polymeric support is said to be mesoporous. It may have a surface area of the pore walls as large as from 200 to 600 m²/g. Preferably, it is in the form of spherical particles. For the solid-state peptide synthesis it is important that the support has a rigid backbone with morphology little dependent on the nature of the surrounding solvent. The peptide synthesis then proceeds in the pore space where the reaction environment can be freely adjusted according to the specific peptide synthesis needs; while in the swollen polymer gel environment in which peptide synthesis proceeds on conventional polymer supports the nature of the polymer supports is always important parameter in the reaction environment control.

For the assessment of the polymeric support morphology, we used inverse steric exclusion chromatography (ISEC) allowing characterization of the polymeric supports under conditions close to their working state, it means in liquid environment (Jeřábek K., Anal Chem. 1984, 57, 1595). Materials re-expanded in tetrahydrofuran were used for the ISEC measurements. This evaluation provides information on the morphology of the investigated material in form of a set of discrete cylindrical pore fractions, each characterized by its volume and diameter. Pore wall surface area S is then computed using formula S = 4v/d, wherein v is the pore volume and d is its diameter.

Porosity is the ratio of the pore volume relative to the total volume of the polymer (share of the pore volume from the total volume of the polymer) and is obtained from ISEC measurements.

The term "medium pore diameter" indicates here the average pore diameter by volume, i.e., an average value of pore diameter weighted by volumes of pore fractions. It is obtained from ISEC measurements.

Surface area of pore walls, referred to herein also as pore wall surface area, is measured by ISEC as described herein above.

The styrenic polymer preferably contains at least 50 mol. % divinylbenzene monomer units. The remaining monomer units may include styrene, ethylvinylbenzene or other monomers, which are typically present in technical grade divinylbenzene.

Said polymeric support can be prepared by radical polymerization from a monomeric mixture comprising at least 50 mol. % divinylbenzene as a crosslinking monomer and a solvent capable of swelling the formed polymer in a volume amount of twice to ten times the volume of the monomer. The solvent is preferably selected from aromatic hydrocarbons, heterocycloalkanes, and halogenated aliphatic hydrocarbons, more preferably from a group consisting of benzene, toluene, xylene, tetrahydrofurane, chloroform, tetrachloromethane, dichloroethane, perchloroethylene. The solvent may preferably comprise up to 20 vol. % of a component which is not capable of swelling styrenic polymers, such component may be selected from liquid alkanes and fatty acids. The preparation of such polymer is described in Czech Pat. Appl. PV 2014-394; and in Hanková L., Holub L., Jeřábek K.: Polym. Sci. Part B-Polym. Phys. 53 (2015)774. Said procedure in highly crosslinked polymers generates pores by a microsyneretic mechanism. In the microsyneretic phase separation mechanism, droplets of liquid (future pores) are expelled from a continuous polymer phase during the polymerization. The microsyneretic pore formation is induced by the high dilution of the monomer(s), more than 50 mol. % of which is the cross-linking component, in a solvent capable of good solvation of the polymer chains during polymerization. The high dilution (2 - 10 volume parts of the solvent per 1 volume part of the monomer(s)) promotes the growth of the polymer chains and disfavors their interconnections. When the overall density of the polymer network is sufficiently high, the phase separation starts, but at this point the polymer network is so extended that the precipitation of solid polymer nuclei cannot occur and segregation of the solvent in the form of excluded droplets takes place instead. As the polymerization process continues, these droplets grow and push the polymer chains close to each other so that cross-linking can now take place in the continuous polymer phase and strengthens the resulting foam-like texture. Within the thus formed porous polymers, the porosity amounts to 60 - 90 % with medium pore diameter in the range of 10 - 80 nm and a surface area of walls of the pores as large as 200 - 600 m²/g. Thanks to the rigid backbone of the polymer, a substantial part of the porosity remains open even after functionalization.
This kind of material offers a high loading capacity for attachment of functional groups using a number of well-known methods, which could serve as sites for anchoring amino acids and peptide chains during the solid-state syntheses. High cross-linking makes the porosity independent of swelling; hence, the morphology of the support is stable regardless of the nature of the surrounding solvent. Therefore, during solid state syntheses on such material, the choice of the reaction solvent is not limited by the nature of the polymer backbone and can be based purely on the demands of the synthetic reaction itself. The pores in the polymer supports proposed by this invention are wider than the spaces inside the swollen polymer gels of the conventional polymer supports for solid-state syntheses, so that they allow the peptide preparation to be free from steric hindrance, even in the case of high molecular weight products. In conventional gel-type polymer supports, the growth of the synthesized molecules stretches the low-crosslinked polymer backbone and hence, the volume of the support particles considerably increases. However, the change of the polymer support volume during the synthesis is undesirable, especially in modern automated synthesis apparatuses using flow-through columns as the reactor. Another advantage of the polymer supports according to this invention is the stability of their particle dimensions during the multi-steps synthesis processes, such as the growth of the synthesized molecules proceeds within constant volume of the support pores.
Styrenic nature of the supports according to this invention allows attachment of a wide variety of peptide synthesis linkers using well established methods. Then, functionalization of the polymer is performed by treatment with a functionalization agent capable of forming functional groups allowing the attachment of amino acids. The functionalization agents include chloromethylation agents, e.g., comprising dimethoxymethane, methanol, acetylchloride, and anhydrous stannic chloride; benzyl-oxy-benzyl-alcoholization agents, e.g., comprising 4-hydroxybenzyl alcohol and sodium methoxide; and/or amination agents such as ethylenediamine.

The present invention further provides a styrenic polymer having porosity 60 - 90 % and medium pore diameter ranging from 10 to 80 nm, preferably having a surface area of the pore walls of 200 to 600 m²/g, said styrenic polymer being functionalized with chloromethyl and/or benzyl-oxy-benzyl alcohol and/or amino moieties. The polymer is preferably in the form of spherical particles.

The present invention also involves use of the functionalized styrenic polymer for solid-state peptide synthesis.

The invention is further illustrated by the following examples, which should not be construed as limiting.

### Examples of carrying out the Invention

EXAMPLE 1. A clear, homogeneous mixture of 6.0 g (6.6 cm³) of divinylbenzene (technical grade, 80%), 60 cm³ of tetrahydrofurane, 6 cm³ of water and 165 mg of the initiator 2,2'-azobis(2-methylpropionitrile) was kept in a closed vessel at 100 °C for 48 hours. After cooling to room temperature, a white, opaque cylinder of relatively soft polymer was removed and dried at room temperature for 10 days. The polymer (hereafter referred to as A) was then ground with pestle in a mortar and dried at 100 °C overnight. Inverse steric chromatography evaluation of its morphology after its re-expansion in tetrahydrofurane showed a pore volume equal to 8.1 cm³/g (corresponds to the porosity 89 %), medium pore diameter (average value weighed by volumes of individual pore fractions) 50 nm and pore wall surface area 640 m²/g.

EXAMPLE 2. A solution of sodium chloride (40 g) and poly(vinyl alcohol) (5 g) in water (600 cm³) was poured into a stainless steel autoclave equipped with a heating mantle, a propeller stirrer and connected to a circulator water bath. A clear, homogeneous mixture of 30.0 g (33 cm³) of divinylbenzene (tech. grade 80%), 240 cm³ of toluene, 60 cm³ of n-heptane and 0.60 g of the initiator 2,2'-azobis(2-methylpropionitrile) as the initiator was added to the aqueous solution inside the autoclave. The autoclave was closed and the content stirred at room temperature for 1 hour. The temperature in the reactor was then raised to 85 °C and kept at this level for 6 hours while the mixture was stirred and for further 48 hours without stirring. After cooling to room temperature the reactor was opened and white polymer beads (hereafter referred to as B) were recovered upon filtration, then washed repeatedly with methanol and dried on air and then in an oven at 100 °C overnight. ISEC evaluation of its morphology after its re-expansion in tetrahydrofuran showed a pore volume of 4.6 cm³/g (considering the skeletal density of the polymer matrix close to 1 g/cm³ it corresponds to the porosity 82 %), medium pore diameter 40 nm and pore wall area 560 m²/g.

EXAMPLE 3. The first step for transforming the mesoporous polymers into supports for the solid state peptide synthesis is their functionalization with chloromethyl groups. In a typical procedure, 22 g of the mesoporous polymer B from Example 2 was suspended in 250 cm³ n-heptane and stirred at room temperature for 1 hour. The reagent for the chloromethylation (110 cm³), which was prepared the day before by mixing of 58 cm³ of dimethoxymethane, 5 cm³ methanol and 47 cm³ acetylchloride, and anhydrous stannic chloride (20 cm³) were added to the suspension. After 15 min the temperature was raised to 40 °C and the reaction let to proceed for another hour. The reaction was quenched by the addition of 200 cm³ of a 1:1 (v:v) mixture of ethanol and water. The product was separated upon filtration and repeatedly washed with ethanol until the filtrate was free of chlorides (negative silver nitrate test). Then it was dried at 105 °C overnight. The final polymer contained 6.7 wt. % chlorine. ISEC evaluation of its morphology after its re-expansion in tetrahydrofuran showed a pore volume of 2.6 cm³/g corresponding to the porosity 72 %, medium pore diameter 39 nm and pore wall area 260 m²/g.

EXAMPLE 4. The chloromethylated styrenic polymer from Example 3 was transformed into the so-called Wang type support, functionalized with 4-benzyloxybenzyl alcohol moieties (hereafter referred to as *pDVB*-4-benzyloxybenzyl alcohol) which is one of possible functionalization useful and desirable for the solid state peptide synthesis. For this purpose 13 g of the styrenic polymer from Example 3 were suspended in 100 cm³ of N,N-dimethylformamide (DMF) and stirred at 65 °C. After 2 hours 6 g of 4-hydroxybenzyl alcohol and 4 g of sodium methoxide were added to the suspension. The mixture was further stirred at the same temperature for 5 hours. After cooling to room temperature the reaction mixture was diluted with methanol and the polymer was recovered upon filtration, thoroughly washed with ethanol and dried at 105 °C overnight. The elemental analysis of the final polymer showed that chlorine dropped from 6.7 wt. % of the polymer from Example 3 to 2.7 wt. %. The mass balance of chlorine indicates that approximately 1.1 mmol/g of the original chloromethyl groups were converted into the desired Wang functionality. ISEC evaluation of its morphology after its re-expansion in tetrahydrofuran showed a pore volume of 2.5 cm³/g corresponding to the porosity 71 %, medium pore diameter 39 nm and pore wall area 256 m²/g.

EXAMPLE 5. The chloromethylated styrenic polymer from Example 3 was transformed in a support functionalized with amino groups (hereafter referred to as pDVB-ethylendiamine), which is another kind of functionalization useful and desirable for the solid state peptide synthesis. For this purpose, the chloromethylated styrenic polymer B from Example 3 (5 g) was suspended in DMF (100 cm³). After 45 min DMF was filtered and the swollen polymer was put in contact with a solution of ethylenediamine (1.26 cm³) dissolved in the smallest amount of DMF required to keep the polymer wet. The reaction was then let to proceed overnight with stirring. The day after, the polymer was washed five times with DMF (50 cm³), then with ethanol and dried at 105 °C overnight. ISEC evaluation of its morphology after its re-expansion in tetrahydrofuran showed a pore volume of 2.6 cm³/g corresponding to the porosity 72 %, medium pore diameter 39 nm and pore wall area 260 m²/g.

EXAMPLE 6. Peptide syntheses were performed according the following procedure: For the attachment of the first amino acid to *pDVB*-4-benzyloxybenzyl alcohol the carboxylic function of the first N-Fmoc protected amino acid (Fmoc-AA-OH) was activated through the formation of a symmetric anhydride. 6 equivalents of Fmoc-AA-OH (with respect to the hydroxyl moiety of *pDVB*-4-benzyloxybenzyl alcohol) were dissolved in the minimum amount of anhydrous DMF and treated with 3 eq (0.11 cm³) N,N'-diisopropylcarbodiimide (DIC). After 30 min the reaction mixture was transferred to a reaction vessel containing the polymer support swollen in DMF, and 0.05 eq (0.001 g) of 4-dimethylaminopyridine was added as a catalyst. The reaction was let to proceed for 1 hour, then the excess of reagents was removed by filtration and the polymer was washed 5-times with DMF and 5-times with dichloromethane (DCM). Deprotection of the amino function of the peptide on the solid support was achieved by a double treatment with a 20 % piperidine solution in DMF, followed by the removal of the excess of reagents by filtration and washing procedure. The coupling of the next incoming Fmoc-AA-OH was performed through the HBTU C-activation method (2-fold excess of Fmoc-AA-OH, N,N,N',N'-tetramethyl-O-(1H-benzotriazol-1-yl)uronium hexafluorophosphate (HBTU) and 1-hydroxybenzotriazole (HOBt); 5-fold excess N,N-diisopropylethylamine (DIPEA), 45 minutes reaction time). The coupling step was followed by a washing procedure. The process was repeated for each new residue in the peptide chain. Once the sequence has been completed, the peptide was cleaved from the polymeric support.

EXAMPLE 7. Synthesis of a model tetrapeptide Fmoc-Leu-Leu-Val-Phe-OH (SEQ ID NO. 1) on *pDVB*-4-benzyloxybenzyl alcohol from Example 4, using acetonitrile as the solvent. To *pDVB*-4-benzyloxybenzyl alcohol from Example 4 Fmoc-Phe-OH was attached as the C-Terminal amino acid and the synthesis of the peptide proceeded according to Example 5, using acetonitrile as the solvent for all the steps of the procedure. The amount of reagents used are reported in Table 1.

**Table 1**

| | | |
|---|---|---|
| *pDVB*-4-benzyloxybenzyl alcohol | | 0.1002 g |
| | | |

| *position in the sequence* | *residue* | *amount* - *g* |
|---|---|---|
| 3 | Val (3eq) | 0.042 |
| 2 | Leu (3eq) | 0.044 |
| 1 | Leu (3eq) | 0.044 |
| For each coupling: | HBTU (3eq) = 0.047g | |
| | HOBt (3eq) = 0.017 g; | |
| | DIPEA (6eq) = 0.042 cm ³ | |

After cleavage from the solid support, using trifluoroacetic acid/triisopropylsilane/water (95:2.5:2.5 v/v), the desired product was obtained with a selectivity of 98%. The same synthesis run on a commercial styrenic Wang resin loaded with Fmoc-Phe-OH, the same amounts of reagents and the same solvent did not get to the target product: the Electron Spray Ionizatio Mass Spectrometry analysis of the products evidenced the presence of truncated and/or deletion sequences.

EXAMPLE 8. pDVB-ethylendiamine from example 5 was suspended in DMF and put in contact with the activated form of Fmoc-Rink linker in presence of HOBt and N-methylmorpholine (NMM). 0.125 g of Fmoc-Rink linker were dissolved in DMF (1.5 cm³) in presence of 2 equivalents of HOBt (0.032 g), 2 equivalents of diisopropylcarbodimmide (DIC) (0.035 cm³) and 2.5 equivalents of NMM (0.025 cm³), the reaction was let to proceed for 4 hours with stirring. The excess of reagents was removed by filtration and the polymer (hereafter referred to as *pDVB*-Rink) washed 5 times with DMF (2 cm³) and further five times with DCM (2 cm³), then dried under vacuum.

The C-terminal residue of the sequence was atteached to *pDVB*-Rink according to the standard procedure for coupling, and the loading of the first residue was determined through the estimation of the Fmoc quantity. The amount of each reagent is reported in Table 2.

**Table 2**

| | | |
|---|---|---|
| *pDVB*-Rink | | 0.115 g |
| | | |

| *C-terminal residue introduction* | | |
|---|---|---|
| | Fmoc-Phe-OH (3eq) | 0.25 g |
| | HBTU (3eq) | 0.24 g |
| | HOBt (3eq) | 0.08 g |
| DIPEA (6eq) | | 0.22 cm³ |
| Measured loading of the first residue | | 0.51 mmol/g |

| *Position in the sequence* | *Residue* | *Amount* - *g* |
|---|---|---|
| 3 | Val (3eq) | 0.059 |
| 2 | Leu (3eq) | 0.062 |
| 1 | Leu (3eq) | 0.062 |
| | | |
| For each coupling: | HBTU (3eq) = 0.067g | |
| | HOBt (3eq) = 0.024 g; | |
| | DIPEA (6eq) = 0.060 cm³ | |

After cleavage from the solid support using trifluoroacetic acid/triisopropylsilane/water (95:2.5:2.5 v/v), the desired product was obtained with a selectivity of 93%.
The same synthesis was carried out using acetonitrile as the solvent, leading to comparable results.

EXAMPLE 9. Synthesis of the fragment [65-74] of the Acyl Carrier Protein **H-Val-Gln-Ala-Ala-Ile-Asp-Tyr-Ile-Asn-Gly-OH** ([65-74]ACP) (SEQ ID NO. 2). The peptide [65-74]-ACP was synthesized on *pDVB*-4-benzyloxybenzyl from Example 4; the C-terminal amino acid glycine was introduced according to Example 5, using DMF as the solvent. The amount of reagents employed are reported in Table 3:

**Table 3**

| | | |
|---|---|---|
| *pDVB*-4-benzyloxybenzyl alcohol | | 0.1994 g |
| | | |

| *Position in the sequence* | *Residue* | *amount* - *g* |
|---|---|---|
| 9 | Asn (4eq) | 0.2009 |
| 8 | Ile (4eq) | 0.1187 |
| 7 | Tyr (4eq) | 0.1544 |
| 6 | Asp (4eq) | 138,26 |
| 5 | Ile (4eq) | 0.1187 |
| 4 | Ala (4eq) | 0.1046 |
| 3 | Ala (4eq) | 0.1046 |
| 2 | Gln (4eq) | 0.2052 |
| 1 | Val (4eq) | 0.1140 |
| | | |
| *For each coupling:* | HBTU (4eq) = 0.127 g; | |
| | HOBt (4eq) = 0.045 g; | |
| | DIPEA (8eq) = 0.115 cm³ | |

The synthesis was run without any optimization of the coupling conditions, nevertheless the desired product was obtained in good yield (43 %) and selectivity (88 %). Similar results were obtained also using acetonitrile as the solvent for all steps of the synthesis on pDVB-4-benzyloxybenzyl alcohol.
The same sequence was prepared also starting from either commercial styrenic Wang resin or Wang resin based on polyethylene glycol. In the first case only a small amount of the target peptide was obtained along with truncated and deletion products while in the latter we got the desired peptide with a good selectivity (85%) but in poor yield (7.5%).

## Claims

1. A method of solid-state synthesis of peptides comprising the steps of
- providing a polymeric solid-state synthesis support,
- attaching a first amino acid to said support to form the first amino acid molecule of the desired peptide chain,
- attaching additional amino acids to form the desired peptide chain,
**characterized in that** the polymeric solid-state synthesis support is a styrenic polymer having a porosity of 60 to 90 % and medium pore diameter of 10 to 80 nm, said styrenic polymer being functionalized with chloromethyl and/or benzyloxybenzyl alcohol and/or amino moieties.

2. The method of claim 1, wherein the styrenic polymer has a surface area of the pore walls 200 to 600 m²/g.

3. The method of claim 1 or 2, wherein the styrenic polymer contains at least 50 mol % divinylbenzene monomer units.

4. The method of any one of the preceding claims, wherein the polymeric solid-state synthesis support is obtainable by a method comprising the step of radical polymerization of a monomeric mixture comprising at least 50 mol % divinylbenzene and a solvent selected from aromatic hydrocarbons, heterocycloalkanes, and halogenated aliphatic hydrocarbons, said solvent being in a volume amount of twice to ten times the volume of the monomer, and said solvent optionally comprising up to 20 vol. % of a component selected from liquid alkanes and fatty acids.

5. A styrenic polymer having porosity 60 to 90 % and medium pore diameter ranging from 10 to 80 nm, said styrenic polymer being functionalized with chloromethyl and/or benzyl-oxy- benzyl alcohol and/or amino moieties.

6. The polymer of claim 5, wherein the styrenic polymer has a surface area of the pore walls from 200 to 600 m²/g.

7. The polymer of claim 5 or 6, wherein the styrenic polymer contains at least 50 mol % divinylbenzene monomer units.

8. Use of the styrenic polymer according to any one of claims 5 to 7 for solid-state peptide synthesis.


SEQUENCE LISTING

<110>  Ustav chemickych procesu AV CR, v. v. i.
       Universita Degli Studi di Padova

<120>  Method of solid-state peptide synthesis using a novel polymeric
       support

<130>  P

<160>  2

<170>  PatentIn version 3.5

<210>  1
<211>  4
<212>  PRT
<213>  artificial

<220>
<223>  model peptide in the example

<400>  1

Leu Leu Val Phe
1

<210>  2
<211>  10
<212>  PRT
<213>  artificial

<220>
<223>  model peptide

<400>  2

Val Gln Ala Ala Ile Asp Tyr Ile Asn Gly
1               5                   10